# EUROPEAN PATENT SPECIFICATION

(11) **EP 1 420 799 B1**
(45) Date of publication and mention of the grant of the patent: **04.10.2006**
(21) Application number: 02797744.6
(22) Date of filing: 21.08.2002
(51) Int. Cl.: A61K 31/7072, A61K 31/551, A61P 31/18

(54) **USE OF ATAZANAVIR IN HIV THERAPY**
VERWENDUNG VON ATAZANAVIR IN DER HIV-THERAPIE
UTILISATION D'ATAZANAVIR EN THERAPIE VIH

(30) Priority: 31.08.2001 US 316745 P
(43) Date of publication of application: 26.05.2004
(73) Proprietor: Bristol-Myers Squibb Company, Princeton, NJ 08543-4000 (US)
(72) Inventor: BECHTOLD, Clifford, M., Guilford, CT 06437 (US)
(74) Representative: Vossius & Partner
(86) International application number: PCT/US2002/026675
(87) International publication number: WO 2003/020206

(56) References cited:
- DATABASE MEDLINE [Online] SANNE ET AL.: 'Results of a phase 2 trial of atazanavir at three doses in combination with didanosine and stavudine in antiretroviral-naive subject', XP002961665 Retrieved from STN Database accession no. 2003007811 & JOURNAL OF ACQUIRED IMMUNE DEFICIENCY SYNDROMES vol. 32, no. 1, 2003, pages 18 - 29
- DATABASE CAPLUS [Online] DE CLERCQ ERIK: 'New anti-HIV agents and targets', XP002961666 Retrieved from STN Database accession no. 2002:891974 & MEDICINAL RESEARCH REVIEWS vol. 22, no. 6, 2002, pages 531 - 565
- E. DE CLERCQ: "New anti-HIV agents and targets" MEDICINAL RESEARCH REVIEWS, vol. 22, no. 6, 2002, pages 531-565,
- K. FORNATARO: "Anti-HIV Drugs: New Findings" BODY POSITIVE, vol. 12, no. 5, May 1999 (1999-05), page 13, NY; USA
- J. GATELL: "Metabolic Toxicities" EUROPEAN CONFERENCE ON CLINICAL ASPECTS AND TREATMENT OF HIV-INFECTION, 24 October 1999 (1999-10-24), pages 1-3,

## Description

This invention relates to the use of atazanavir for reducing certain metabolic side effects in HIV-infected patients, said side effects possibly resulting from the administration of one or more HIV protease inhibitors. More particularly, the invention relates to the substitution of the HIV protease inhibitor, atazanavir, for an HIV protease inhibitor causing elevated blood LDL and/or triglyceride levels or the combination of atazanavir with such other HIV protease inhibitor.

Combination antiretroviral therapy that includes protease inhibitors represents a significant advance in the therapy of HIV infection. HIV protease inhibitors may be combined with HIV inhibitors of other classes, e.g. nucleoside reverse transcriptase inhibitors or non-nucleoside reverse transcriptase inhibitors, or used with other HIV protease inhibitors, often also with nucleoside reverse transcriptase inhibitors and/or non-nucleoside reverse transcriptase inhibitors, in so-called "cocktails". Combinations of protease inhibitors have been suggested to combat viral resistance (see, for example, U.S. 6,100,277). The protease inhibitor, ritonavir, has also been disclosed as improving the pharmacokinetics of certain other protease inhibitors when used in combination (see, for example, U.S. 6,037,157).

A number of HIV protease inhibitors are on the market or under clinical investigation including saquinavir, indinavir, ritonavir, nelfinavir, amprenavir, atazanavir, tipranavir, and lopinavir. Atazanavir (also referred to as BMS-232632) is disclosed in U.S. Patents 5,849,911 and 6,166,004. U.S. Patent 6,087,383 discloses the crystalline bisulfate salt of atazanavir. Atazanavir is an azapeptide inhibitor currently in development and undergoing clinical evaluation. It has high potency, a favorable resistance profile, and bioavailability supportive of once-daily oral dosing.

Administration of HIV therapy containing HIV protease inhibitors has been found to be accompanied by certain metabolic abnormalities in a significant number of patients, including an elevation in plasma concentrations of low-density lipoproteins (LDL) and triglycerides. Elevation in plasma levels of LDL-cholesterol and triglycerides constitutes a major risk factor for the premature development of atherosclerosis and hypertriglyceridemia, and may also contribute to the development of heart disease and pancreatitis. Individual protease inhibitors differ in their effects on serum lipid profiles, but in general, the current experience is that, to date, all such inhibitors have exhibited this metabolic liability. Ritonavir seems to have the greatest effect, while indinavir, nelfinavir, amprenavir, and saquinavir appear to have somewhat reduced effects on serum lipid levels. The increased LDL-cholesterol and triglyceride levels associated with protease inhibitor use are of growing concern, particularly when regimens containing two or more protease inhibitors are employed.

The frequency of hyperlipidemia in HIV-infected patients taking protease inhibitors has been reported to range from 8% to as high as 66% (see *Pharmacotherapy,* 1999, 19:281-298).

One suggested solution to the above problem has been to administer antilipidemic agents to patients having elevated plasma lipid levels resulting from HIV protease inhibitor therapy. However, the most commonly used antilipidemic agents, hydroxymethylglutaryl-coenzyme A (HMG-CoA) reductase inhibitors, or statins, are metabolized by the cytochrome P450 isoenzyme system. Thus, at least with some statins such as simvastatin and lovastatin, antihyperlipidemic efficacy in HIV-infected patients on protease inhibitor therapy may be compromised at standard statin doses.

In the journal BODY POSITIVE **1999,** *XII* (5) and on the EUROPEAN CONFERENCE ON CLINICAL ASPECTS AND TREATMENT OF HIV-INFECTION (October 24, 1999) it was reported that HIV treatment with protease inhibitors has as a side effect elevated LDL and/or triglyceride levels. As a countermeasure, administration of an antilipidemic drug, or substituting, at least partially, the protease inhibitor with a non-protease inhibitor was suggested.

In one aspect, the present invention relates to the use of atazanavir for the manufacture of a medicament for reducing elevated plasma LDL-cholesterol and/or triglyceride levels in an HIV-infected patient undergoing therapy with one or more HIV protease inhibitors resulting in such elevated LDL-cholesterol and/or triglyceride levels, wherein the medicament is adapted for substituting in such therapy an HIV-inhibiting and LDL-cholesterol and/or triglyceride reducing amount of atazanavir for the offending HIV protease inhibitor. The reduction in hyperlipidemia is similar to that achieved by use of a statin, but without the side effects seen with this class of lipid-lowering agents. In another aspect, the present invention relates to the use of atazanavir for the manufacture of a medicament for reducing elevated plasma LDL-cholesterol and/or triglyceride levels in an HIV-infected patient undergoing HIV protease inhibitor therapy which comprises administering to said patient an effective HIV-inhibiting amount of atazanavir in combination with an HIV-inhibiting amount of at least one other HIV protease inhibitor which is metabolized by cytochrome P450 monooxygenase. In yet another aspect, the present invention relates to the use of atazanavir for the manufacture of a medicament for treating HIV infection in a patient exhibiting elevated plasma LDL-cholesterol and/or triglyceride levels.

The present invention is based on the unexpected observation that atazanavir, unlike other HIV protease inhibitors, has no significant effect on plasma LDL-cholesterol and triglyceride levels when administered in normal HIV-inhibiting dosages.

In clinical studies carried out on 98 HIV-positive patients who have taken atazanavir for one year, no increases in plasma LDL-cholesterol or triglycerides were observed. Thus, atazanavir is particularly useful in the treatment of HIV-positive patients who have elevated plasma LDL-cholesterol and/or triglyceride levels. It can be used as monotherapy or as part of a "cocktail" which would include other antiretroviral drugs such as reverse transcriptase inhibitors, non-nucleoside reverse transcriptase inhibitors, and other HIV protease inhibitors. In one aspect, then, the present invention relates to the use of atazanavir for the manufacture of a medicament for treating HIV infection in a patient exhibiting elevated plasma LDL-cholesterol and/or triglyceride levels

Atazanavir is especially useful in the treatment of HIV-infected patients who have elevated plasma LDL-cholesterol and/or triglyceride levels resulting from antiretroviral therapy with an HIV protease inhibitor.

In a Phase II randomized study, the safety and anti-HIV activity of three once daily doses of atazanavir (200 mg, 400 mg and 500 mg) was compared with 750 mg of the protease inhibitor, nelfinavir dosed three times daily. Twenty-one patients received nelfinavir in combination with stavudine and didanosine, while atazanavir was administered both as monotherapy (for two weeks) and then in combination with stavudine and didanosine to 78 treatment-naive (no prior anti-HIV therapy) patients with HIV viral loads >2000 copies per milliliter (c/mL). Patients in the atazanavir arms showed at least comparable reductions in viral load compared to the nelfinavir-treated patients, but displayed no changes in lipid levels, while after four weeks, those in the nelfinavir arm had an increase in total cholesterol, LDL-cholesterol and triglyceride levels.

In another two-stage, randomized three-arm Phase II study, the antiviral activity and metabolic effects of atazanavir at two different doses (400 mg or 600 mg) was compared to ritonavir, each in combination with saquinavir over 48 weeks. The protease inhibitors were administered in combination with two nucleoside reverse transcriptase inhibitors selected from didanosine, stavudine, lamivudine or zidovudine. The selection of reverse transcriptase inhibitors (used in combination as didanosine + stavudine, stavudine + lamivudine, didanosine + zidovudine, or lamivudine + zidovudine) was based on the patient's phenotypic sensitivity and the physician's choice at the time of administration. The regimens were as follows:
atazanavir 400 mg QD + saquinavir 1200 mg QD + 2 nucleoside reverse transcriptase inhibitors
atazanavir 600 mg QD + saquinavir 1200 mg QD + 2 nucleoside reverse transcriptase inhibitors
ritonavir 400 mg BID + saquinavir 400 mg BID + 2 nucleoside reverse transcriptase inhibitors

Patients were anti-retroviral-experienced with a treatment history of receiving a minimum of 24 weeks of a protease inhibitor-containing or non-nucleoside reverse transcriptase inhibitor-containing combination regimen. Stage 1 was to randomize approximately 75 subjects to assess the safety and antiviral activity of the regimens. Following an initial safety assessment of Stage I, an additional 300 patients were randomized into Stage II to assess the magnitude and duration of reduction of plasma HIV RNA from baseline through week 48. The two atazanavir groups were compared to ritonavir in terms of the mean change in total cholesterol from baseline at week 12. The difference estimates for the pairwise comparisons were -49.1 for the atazanavir 600 mg -ritonavir pair and -28.7 for the atazanavir 400 mg-ritonavir pair. Total cholesterol was not elevated on either of the atazanavir groups. The median changes from baseline at week 12 were -20 mg/dL for atazanavir 600 mg and 5 mg/dL for atazanavir 400 compared to 37 mg/dL for ritonavir. Fractionated cholesterol data were available at baseline and week 12 on approximately ½ of the patients. LDL-cholesterol was not elevated over baseline on any atazanavir group.

The two atazanavir groups were compared to ritonavir in terms of the mean change in triglycerides from baseline at week 12. The difference estimates for the pairwise comparisons were -135.9 for atazanavir 600-ritonavir and-155.2 for atazanavir 400 mg-ritonavir. The median changes from baseline at week 12 were 5 mg/dL for atazanavir 600 mg and -14 mg/dL for atazanavir 400 mg compared to 102 mg/dL for ritonavir. The two groups showed similar antiviral efficacy through week 12. This study shows that patients with elevated plasma LDL-cholesterol and triglycerides can be effectively switched to an atazanavir-containing therapy to maintain viral suppression while reducing LDL-cholesterol and triglyceride levels. Thus, the present invention provides an effective and safe alternative to statin intervention that can result in additional side effect risks and adversely impact other medications.

Atazanavir can be used alone or in combination with one or more other pharmaceutically active substances that are active against retroviruses, especially HIV. Such other substances may be, for example, inhibitors of reverse transcriptase, e.g. zidovudine, didanosine, stavudine, zalcitabine, or lamivudine, non-nucleoside reverse transcriptase inhibitors such as efavirenz, or other HIV protease inhibitors.

Atananivir is normally obtained as a weak organic base and is commonly converted for pharmaceutical formulation into pharmaceutically acceptable acid addition salts such as those described in U.S. Patent 6,166,004. A most preferred salt form for pharmaceutical compositions is the bisulfate salt described in U.S. Patent 6,087,383.

In the use of atazanavir according to the present invention, one starts with an HIV-infected patient who has elevated plasma LDL-cholesterol and/or triglyceride levels. In one aspect of the invention, such patient has been or is undergoing antiretroviral therapy with one or more HIV protease inhibitors other than atazanavir and exhibits elevated plasma LDL-cholesterol and/or triglyceride levels. Such other HIV protease inhibitor(s) may be given as monotherapy or as part of an antiretroviral therapy which also includes one or more other antiretroviral drugs such as reverse transcriptase inhibitors or non-nucleoside reverse transcriptase inhibitors. Such candidates, although they may exhibit satisfactory viral suppression, may be of increased risk for hyperlipidemia and premature cardiovascular events.

The term "elevated plasma LDL-cholesterol and triglyceride levels" as used herein is based on the National Cholesterol Education Program (NCEP) clinical practice guidelines for the prevention and management of high cholesterol in adults. In the latest guidelines issued in 2001, plasma levels of >130 mg/dL of LDL-cholesterol and >150 mg/dL of triglycerides are considered elevated or "high". The process of the present invention is particularly useful for those patients having plasma triglyceride levels of >200 mg/dL and for those patients with no risk factors or previous cardiovascular events having LDL-cholesterol levels of >160 mg/dL. The definition of "elevated" LDL-cholesterol and triglyceride levels may, of course, change in the future as the NCEP continues to evaluate heart attack risk factors. It is intended, then, that the term "elevated LDL-cholesterol and triglyceride levels" as used will be consistent with current NCEP guidelines.

In one of its aspects, the present invention involves discontinuing the offending (the drug responsible for the elevated plasma LDL-cholesterol and/or triglyceride levels) HIV protease inhibitor from the above regimen and substituting therefore an amount of atazanavir which is effective to inhibit HIV and to reduce plasma LDL-cholesterol and/or triglyceride levels.

The dose of atazanavir to be employed depends on such factors as the body weight, age and individual condition of the patient to be treated and the mode of administration. In general dosages of from about 3 mg to approximately 1.6 grams per person per day, divided into 1 to 3 single doses, are suitable. An especially preferred dosage for adult patients is 50-800, more preferably 400-600 and most preferably 400 mg given once daily.

The pharmaceutical compositions of atazanavir that may be used in the present invention are those described, for example, in U.S. Patents 6,166,004 and 6,087,383. Suitable forms for oral administration include capsules, tablets and powders for oral suspension.

Atazanavir has also been found to be an inhibitor of cytochrome P450 monooxygenase and can improve the pharmacokinetics of drugs metabolized by this enzyme, including particularly other HIV protease inhibitors such as saquinavir, indinavir, nelfinavir, amprenavir, tipanavir and lopinavir. Thus, it acts in a similar way to ritonavir described in U.S. Patent 6,037,157 to increase blood levels of the co-administered HIV protease inhibitor. One notable difference from ritonavir is that atazanavir is employed in combination therapy with other HIV protease inhibitors at its normal therapeutic dose level instead of the sub-therapeutic dose levels used with ritonavir. Because of its potentiating effect on other HIV protease inhibitors which are metabolized by cytochrome P450 monooxygenase, use of atazanavir concomitantly with such other HIV protease inhibitors allows reduced dosages of such other HIV protease inhibitors to be used while maintaining the same degree of viral suppression. For example in the Phase II study described above, the dosage of saquinavir could be adjusted from its recommended dosage of 1200 mg 3 times daily to 1200 mg once daily when combined with atazanavir. Atazanavir can be used in combination with other HIV protease inhibitors to reduce LDL-cholesterol and triglyceride levels in AIDS patients undergoing protease inhibitor therapy while still maintaining the desired level of viral suppression.

The appropriate dose of the HIV protease inhibitor being combined with atazanavir can be determined by the following method which was used for the atazanavir/saquinavir combination.

Atazanavir is a moderate inhibitor of the cytochrome P450 3A enzyme comparable to nelfinavir and indinavir, with a Kᵢ of 2.4 µM. The latter two compounds increase the exposure of saquinavir (dosed at 1200 mg thrice-daily (TID) by 392 and 364%, respectively, at steady-state. A multiple-dose pharmacology study was completed to evaluate if a similar increase could be expected for the combination of atazanavir and saquinavir. This study showed a greater than 3-fold increase in exposure, due to combination with atazanavir, supporting a 1200 mg once-daily saquinavir dosing, was equivalent to the currently marketed saquinavir regimen of 1200 mg TID. Using a constant dose of atazanavir the range of saquinavir doses were studied to target the saquinavir exposure (AUC (area under the curve) and CMIN (minimum concentration)) similar to those in the literature. Similarly, appropriate dosing of other HIV protease inhibitors to be used in combination with atazanavir can be calculated.

In another aspect, then, the present invention relates to the use of atazanavir for the manufacture of a medicament for reducing plasma LDL-cholesterol and/or triglyceride levels in an HIV-infected patient undergoing HIV protease inhibitor therapy which comprises administering to said patient an effective HIV-inhibiting amount of atazanavir in combination with an HIV-inhibiting amount of at least one HIV protease inhibitor metabolized by cytochrome P450 monooxygenase.

The present invention also provides pharmaceutical compositions comprising an effective HIV-inhibiting amount of atazanavir and an effective HIV-inhibiting amount of at least one other HIV protease inhibitor which is metabolized by cytochrome P450 monooxygenase. Compositions in the form of capsules, tablets, nasal sprays, solutions, powders, granules, emulsions, syrups, suppositories, liposomes, suspensions, etc. such as those disclosed in U.S. Patent 6,166,004 are suitable for the present invention.

## Claims

1. Use of atazanavir for the manufacture of a medicament for reducing elevated plasma LDL and/or triglyceride levels in an HIV-infected patient resulting from therapy with one or more HIV protease inhibitors, the medicament being adapted for substituting an HIV-inhibiting and LDL and/or triglyceride reducing amount of atazanavir for the offending HIV protease inhibitor used in such therapy.

2. Use of atazanavir for the manufacture of a medicament for reducing elevated plasma LDL and/or triglyceride levels in an HIV-infected patient undergoing HIV protease therapy which comprises administering to said patient an effective HIV-inhibiting amount of atazanavir in combination with an HIV-inhibiting amount of at least one HIV protease inhibitor metabolized by cytochrome P450 monooxygenase.

3. The use of Claim 2 wherein atazanavir is administered in combination with an HIV protease inhibitor selected from saquinavir, indinavir, amprenavir, nelfinavir, tipanavir or lopinavir.

4. The use of Claim 3 wherein atazanavir is administered in combination with saquinavir.

5. Use of atazanavir for the manufacture of a medicament for treating HIV infection in a patient exhibiting elevated plasma LDL-cholesterol and/or triglyceride levels.

## Patentansprüche

1. Verwendung von Atazanavir für die Herstellung eines Arzneimittels zum Verringern erhöhter Plasma-LDL- und/oder -Triglycerid-Spiegel in einem HIVinfizierten Patienten, die von Therapie mit einem oder mehreren HIV-Protease-Inhibitoren herrühren, wobei das Arzneimittel an das Austauschen einer HIV-hemmenden und LDL- und/oder Triglycerid-verringernden Menge von Atazanavir für den angreifenden HIV-Protease-Inhibitor, der in einer solchen Therapie verwendet wird, angepasst ist.

2. Verwendung von Atazanavir für die Herstellung eines Arzneimittels zum Verringern erhöhter Plasma-LDL- und/oder -Triglycerid-Spiegel in einem HIVinfizierten Patienten, der einer HIV-Protease-Therapie unterzogen wird, umfassend das Verabreichen an den Patienten einer wirksamen HIV-hemmenden Menge von Atazanavir in Kombination mit einer HIV-hemmenden Menge von mindestens einem HIV-Protease-Inhibitor, der durch Cytochrom P450-Monooxygenase metabolisert wird.

3. Verwendung gemäß Anspruch 2, wobei Atazanavir in Kombination mit einem HIV-Protease-Inhibitor, ausgewählt aus Saquinavir, Indinavir, Amprenavir, Nelfinavir, Tipanavir oder Lopinavir, verabreicht wird.

4. Verwendung gemäß Anspruch 3, wobei Atazanavir in Kombination mit Saquinavir verabreicht wird.

5. Verwendung von Atazanavir für die Herstellung eines Arzneimittels zur Behandlung einer HIV-Infektion in einem Patienten, der erhöhte Plasma-LDL-Cholesterol- und/oder -Triglycerid-Spiegel aufweist.

## Revendications

1. Utilisation d'atazanavir pour la fabrication d'un médicament pour réduire des niveaux élevés de LDL et/ou de triglycérides dans le plasma chez un patient infecté par VIH, résultant d'une thérapie avec un ou plusieurs inhibiteurs de protéase de VIH, le médicament étant adapté à substituer une quantité d'inhibition de VIH et de réduction de LDL et des triglycérides d'atazanavir à l'inhibiteur de protéase de VIH offensif utilisé dans une telle thérapie.

2. Utilisation d'atazanavir pour la fabrication d'un médicament pour réduire des niveaux élevés de LDL et/ou de triglycérides dans le plasma chez un patient infecté par VIH subissant une thérapie par la protéase de VIH, qui consiste à administrer audit patient une quantité efficace d'inhibition de VIH d'atanazavir en combinaison avec une quantité d'inhibition de VIH d'au moins un inhibiteur de protéase de VIH métabolisé par la cytochrome P450 monooxygénase.

3. Utilisation de la revendication 2, où l'atazanavir est administré en combinaison avec un inhibiteur de protéase de VIH sélectionné parmi saquinavir, indinavir, amprenavir, nelfinavir, tripanavir ou lopinavir.

4. Utilisation de la revendication 3, où l'atazanavir est administré en combinaison avec le saquinavir.

5. Utilisation d'atazanavir pour la fabrication d'un médicament pour le traitement d'une l'infection par VIH chez un patient présentant des niveaux élevés de cholestérol LDL et/ou de triglycérides dans le plasma.
